# EUROPEAN PATENT SPECIFICATION

(11) **EP 1 985 328 B1**
(45) Date of publication and mention of the grant of the patent: **17.10.2012**
(21) Application number: 07008705.1
(22) Date of filing: 27.04.2007
(51) Int. Cl.: A61M 16/20, F16K 47/00

(54) **Sound absorber and method for absorbing sound**
Schallabsorber und Verfahren zum Absorbieren von Schall
Amortisseur de bruit et procédé d'absorption du bruit

(43) Date of publication of application: 29.10.2008
(73) Proprietor: MAP Medizin-Technologie GmbH, 82152 Martinsried (DE)
(72) Inventor: Burz, Johann S., 82407 Wielenbach/Wildzhofen (DE); Lang, Bernd, 82166 Gräfelfing (DE)
(74) Representative: Vossius & Partner

(56) References cited:
- WO-A-02/15969
- JP-A- 2003 190 238
- US-A- 4 699 137
- US-A- 6 073 630

## Description

The present invention relates to a method and device for absorbing sound, particularly in the fields of respiratory devices and particularly for absorbing the sound of an expiration valve of a ventilation means such as a respiratory device.

In the field of supported ventilation, such as invasive (IV) or non-invasive (NIV) ventilation, single hose systems and systems having two separate hoses, such as an inspiration and expiration branch, are known.

In single hose systems, generally one hose is provided between a ventilation means, such as a breathing device, and a breathing mask for a patient. Near the mask, or even integrated in the mask, is a so-called leakage or expiration opening through which exhaled air or expiration gas continuously leaks or escapes. A number of approaches are known in the art to make this leakage as noiseless as possible.

Systems having an expiration branch and an inspiration branch are generally provided with an expiration valve being provided in the expiration branch and being controlled, e.g., by the ventilation means. By means of this expiration valve, the expiration branch can be controlled to release a volume of exhaled air or expiration gas to the ambience. These so-called expiration valves often create disturbing noises during operation and particularly when being opened and/or when releasing exhaled air.

In the art, it is known to provide the expiration valve either close to the mask/patient or to arrange it close to the breathing device or even in the breathing device via a second hose or expiration branch. The latter systems are, e.g., also commonly used in invasive ventilation.

US-A-6073630 refers to a reciprocating pump particularly useful in ventilator apparatus including a piston reciprocatable axially_within a cylinder and dividing its interior into an inlet chamber and an outlet chamber, a wall fixed within the inlet chamber, and a drive housing fixed to the wall. The drive housing includes a motor, a rotor rotatable bv the motor, a nut rotatable within the drive housing, and a screw threadedly coupled at one end to the nut and fixed at its opposite end to the piston. The piston is substantially unrestrained for axial and rotary movement such that forward and reverse rotation of the nut by the motor reciprocates the screw and the piston axially of the cylinder, and also permits the screw and the piston to rotate with respect to the cylinder to thereby even out wear between the pistol and the cylinder.

US-A-4699137 refers to an exhalation valve having an inlet, an outlet and a diaphragm movable between closed position covering the inlet and an open position away from the inlet. The valve includes a bias means comprising a sealed chamber containing a gas acting upon the diaphragm and urging the diaphragm to its closed position. An openings means in said sealed chamber conducts a control pressure to chamber to create the bias acting against one side of the diaphragm. The diaphragm comprises a flexible peripheral portion and a semi-rigid plunger. The plunger has a projection adapted to move within a confined space to align the diaphragm and provide damping to its movement. The flexible peripheral portion of the diaphragm has a specially shaped cross-section comprising two attached cantilever sections that reduce the effective resistance to motion between open and closed positions. In the preferred embodiment, the plunger is an insert that fits into and its retained by the flexibly peripheral portion.

In the field of invasive, tubular ventilation the patient is usually sedated in order to accept or tolerate the tubus. For those patients, the level of noise of a ventilation means is of minor importance since their consciousness and capability of sensing noise is reduced if at all present. However, the staff, e.g., at the intensive care station in a hospital may be disturbed by the sound of the ventilation means thereby negatively affecting the quality of work.

In fields where breathing of a conscious patient is assisted, patients themselves are affected by the noise created by the ventilation means or breathing device. In particular, when a patient's breathing is assisted during sleep of the patient, such noise is considered as being unpleasant and causing discomfort thus affecting the patient's physical and psychological constitution.

Accordingly, there is an object underlying the present invention to increase the comfort of a patient as well as of medical staff and/or other people living with a patient using ventilation means and being dependent on assisted breathing with regard to noise pollution or noise disturbance.

It has been found out that conducted noise is transported from the ventilation means of breathing devices via the breathing hose to the patient. Additionally, a disturbing noise is radiated to the ambience.

Moreover, it has been found out that also the operation of the expiration valve used in breathing devices for releasing patient's exhaled breath to the ambience is a source of undesired noise contributing to a patient's discomfort and the like. Also this sound or noise created by the expiration valve is conduced to the patient mainly via the respirations hose.

The present invention provides a sound absorber or muffler as well as a method for absorbing sound of a breathing device or ventilation means, particularly the sound as emitted by an expiration valve of a ventilation means or breathing device.

Moreover, the present invention relates to an expiration valve and sound absorber for breathing devices.

The object underlying the present invention is achieved by the features of the independent claims. The dependent claims define further preferred embodiments according to the present invention.

According to a preferred embodiment of the present invention, there is provided a sound absorber or muffler for ventilation means, particularly for breathing devices having an expiration valve, comprising a first flow channel and a second flow channel for conducting breathable air or gas. The first and second flow channels extend substantially coaxial to one another and a ventilation means or an expiration valve is connectable to the first flow channel and the second flow channel so that both air flowing to the expiration valve and air flowing from the expiration valve is directed through one of the first and second flow channels of the sound absorber or muffler. In particular, air flowing to the expiration valve flows through the first or second flow channel whereas air flowing from the expiration channel flows through the other flow channel.

Both, coextending first and second channels are located in one housing providing a sound absorbing space or volume.

According to a preferred embodiment, there is provided a sound absorber or muffler, preferably for damping conducted noise, comprising a first connector for connection to an expiration conduct of a breathing device and, a second and third connector for connection to an inspiration conduit of the breathing device, and a fourth connector for connection to an expiration valve of the breathing device. The first and fourth as well as the second and third connector are preferably located at opposing ends of the sound absorber so that the flow of breathing (inspiration and expiration) air is both directed through the sound absorber. Moreover, also the air released to the ambience by the expiration valve is directed through the sound absorber wherein the expiration valve is located such that the flow of expiration air is firstly conducted through the sound absorber to the expiration valve and then again through the sound absorber to the ambience.

Preferably, the first and second flow channels connect the first and the second as well as third and fourth connectors of the sound absorber. Even more preferably, the first and second flow channels are designed as two coaxially extending tubes.

According to a preferred embodiment, the first channel is located inside the second channel or the second channel extends around the first channel. Preferably, the first channel is for conducting exhaled air to the expiration valve and the second channel is for conducting exhaled air from the expiration valve, preferably to the ambience.

The sound absorbing volume is preferably filled with a sound absorbing medium such as air, gas, liquid, fluid, steel, wool, sintered materials, porous materials, foam or foamed materials, non-woven materials and woven materials and/or knitted fabrics and the like.

Preferably, the first and second flow channels are distinct from one another and preferably are separated by a tubular separation wall. The second flow channel is preferably defined by the separation wall which also defines the first flow channel and a sound absorbing volume provided by a sound absorber housing. The sound absorber according to the present invention is particularly suitable for being connected in the expiration line or conduit of a breathing device or ventilation means wherein the sound absorber has one connection means for being connected to the inlet and the outlet of an expiration valve so that the air or gas flowing through the expiration valve is directed through a first conduit or channel of the sound absorber and whereas the air or gas emitted from the expiration valve is directed through a second channel or conduit of the sound absorber. Preferably, the first and second channels of the sound absorber are arranged such, that at the position of the connection means, the cross-section or opening of the first conduit is of basically round or circular shape, whereas the cross-section or opening of the first channel is of generally ring-like shape surrounding the circumference of the opening of the second channel. According to further preferred embodiments, these respective cross-sections or openings may be of other shapes than circular, e.g., rectangular or the like.

According to a further preferred embodiment, the sound absorber further comprises a third flow channel for conducting inspiration air from a ventilation means or breathing device to a patient.

Accordingly, in this embodiment the sound absorber comprises one channel for directing gas to the expiration valve and one channel for releasing air from the respiration valve to the ambience. Thus, as discussed above, the sound absorber comprises one opening towards the expiration line of the breathing system as well as two, preferably coaxial, openings for connection to the expiration valve and one opening to the ambience. Furthermore, two additional openings for connection to the respective conduit portions of the inspiration line, i.e., one for connection to the inspiration line and one for connection to the ventilation means; in other words one opening directed towards the patient and one directed towards the ventilation means.

According to a preferred embodiment, the first and/or third channels of the sound absorber are constituted by the expiration and inspiration hose, respectively, of the breathing device. In this case, the housing of the sound absorber surrounds the expiration hose and/or inspiration hose, thereby providing a sound absorbing volume as discussed above.

Accordingly, as is apparent from the above discussion, the present invention provides an improved sound absorber or muffler wherein one preferred embodiment relates to an improved sound absorber for an expiration valve being located inside the ventilation means or breathing device and wherein a further preferred embodiment relates to an improved sound absorber or muffler for an expiration valve being located outside the ventilation means or breathing device. According to a further preferred embodiment, the sound absorber additionally damps the conducted noise conducted from a ventilation means or breathing device via the inspiration line.

According to a preferred embodiment, the sound absorber is integrated in breathing device or ventilation means.

As is also apparent from the above discussion, the basic concept underlying the preferred embodiments of the present invention are the same, as will be also apparent from the following detailed discussion.

The sound absorber according to the present invention is particularly advantageous in that it significantly reduces the noise made by the operation of the expiration valve of a ventilation means thereby improving the comfort of a patient or user using the breathing device or ventilation means. Moreover, the co-axial extension of the respective conduit according to preferred embodiments of the present invention is particularly advantageous in that it contributes to small sizing of the sound absorber without deteriorating its performance.

Further detailed and/or preferred embodiments of the present invention will become apparent from the following exemplary description with reference to the Figures. Of these Figures,
- Fig. 1: shows a cross-sectional view of a sound absorber according to a first embodiment of the present invention,
- Fig. 2: shows a cross-sectional view of a sound absorber according to a second embodiment of the present invention,
- Fig. 3: shows an exploded view of a preferred embodiment of a sound absorber according to the present invention,
- Fig. 4: shows a further schematic sectional view of a sound absorber according to a preferred embodiment of the present invention, and
- Fig. 5: shows a schematic three-dimensional view onto sound absorber 1 according to a preferred embodiment of the present invention from a first side (Figure 5a) and from a second side (Figure 5b).

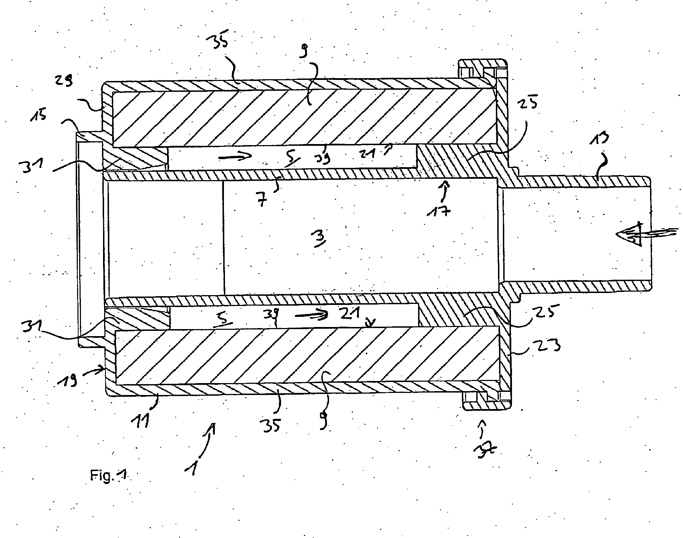

Fig. 1 shows a preferred embodiment of the sound absorber according to the present invention. Sound absorber 1 comprises a first conduit 3 and a second conduit 5 which are coaxially arranged and which are separated by a tubular separation wall 7. A sound absorbing volume 9 of the sound absorber 1 having an optional filling, preferably a porous foam or an open cell foam material, is provided in a housing 11 surrounding the first and the second channels 3, 5.

The inner channel 3 is defined by a separation wall 7. However, according to further or additional preferred embodiments, the inner channel 3 may also be defined by an expiration hose of a breathing conduit. In this case, preferably, the separation wall 7 is substituted by the walls of the expiration hose.

The sound absorber 1 comprises a connector 13 arranged at end of the sound absorber directed towards the patient for connecting and/or for receiving an expiration hose of a breathing system. The sound absorber 1 further comprises a second connector 15 for direct or indirect connection to an expiration valve (not shown). Preferably, connector 15 is arranged at the side of the sound absorber facing towards the ventilation means or breathing device, i.e., preferably at the side opposite to the side on which the connector 13 is provided.

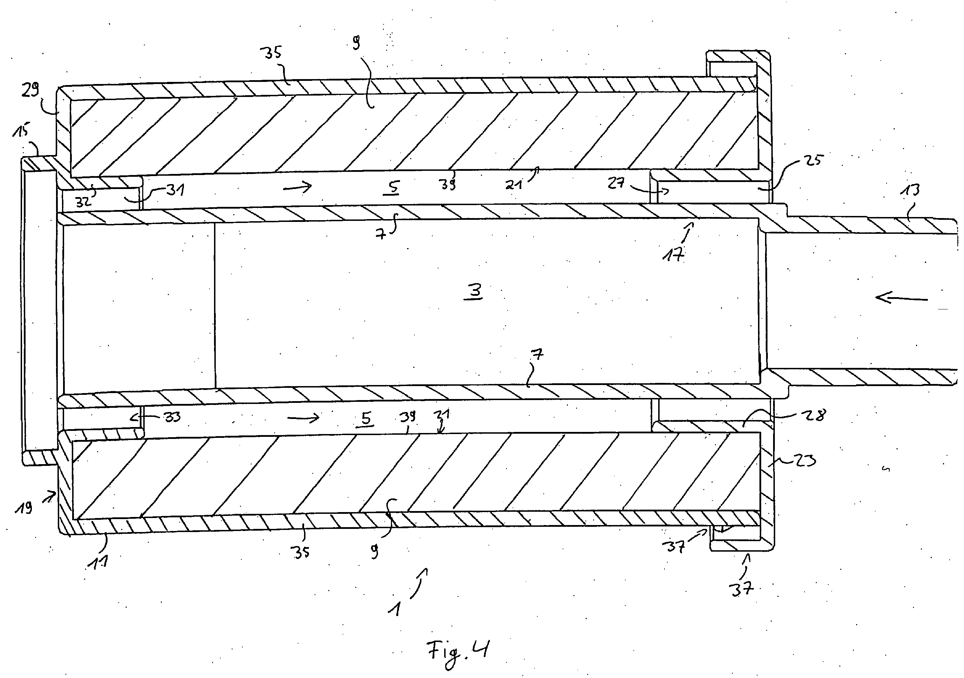

During use, air exhaled by the patient is conducted through the expiration hose to the sound absorber 1 and flows through the channel 3 to the expiration valve (not shown). The expiration valve is operated to release the exhaled air into channel 5, and thus again through the sound absorber 1. The exhaled air is then released to the ambience at the opening 27 of channel 5 surrounding connector 13 (not visible in the cross-sectional view shown in Fig. 1; see Fig. 4).

Thus, since the sound absorber is arranged downstream of the expiration branch of the breathing system and expirated air only flows through the sound absorber in one direction, a possible contamination (e.g., with germs) is, generally not considered as being problematic.

Preferably, volume 9 or the respective filler medium coming into contact with the exhaled air can easily be exchanged due to a preferred modular assembly of the sound absorber to be described later.

In order not to influence the expiration pattern or behavior of the patient, the sound absorber preferably has minimal flow resistance. According to preferred embodiments, other known techniques for absorbing the sound, such as by the use of the sintered or paper cartridges, are used to realize the sound absorber according to the present invention.

Needless to say that a sound absorber 1 located downstream of a separate expiration valve also serves to reduce or damp the noise generated by the expiration valve. The parallel or coaxial arrangement of channels 3 and 5 particularly contributes to downsize the sound absorber.

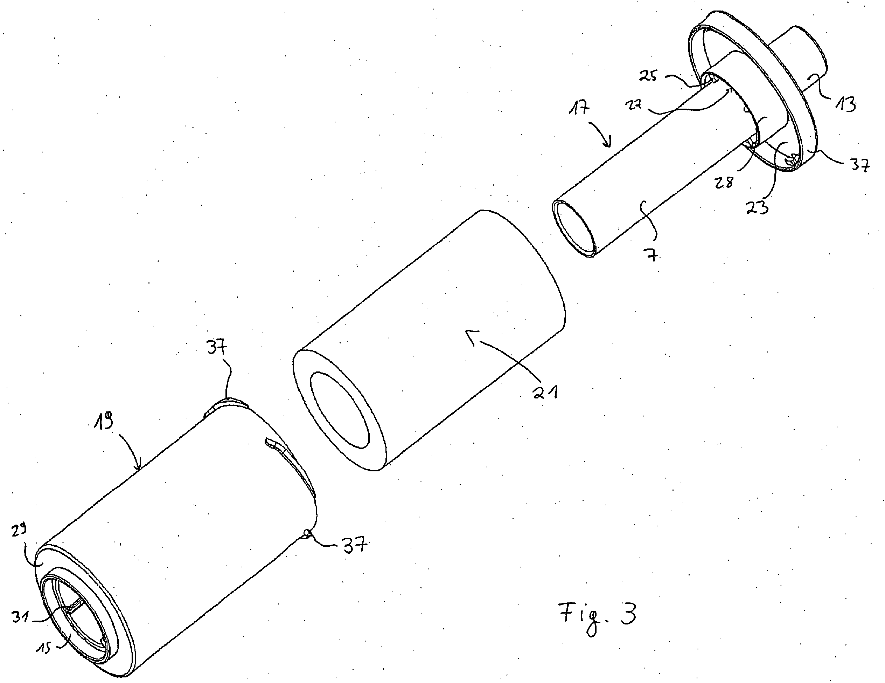

According to a preferred embodiment, as e.g., schematically shown in Figs. 1 and 3, sound absorber 1 generally comprises three main structural components. Preferably, sound absorber 1 comprises an inner channel module 17, an outer channel module 19 and, optionally, a sound absorbing medium component 21.

Preferably, the inner channel module 17 comprises a portion constituting a separation wall 7 thereby defining an inner channel 3 as well as a connecting portion 13, as discussed above. Inner conduit portion 17 furthermore preferably comprises a lid or sidewall portion 23 for forming a sidewall of the sound absorber. Preferably, sidewall 23 is arranged close to the point of connection of separation wall 7 and connector 13. Inner channel module 17 furthermore comprises ridges 25 arranged at the circumference of separation wall 7, preferably adjacent wall 23. Preferably, ridges 25 are equally distributed or distributed in a star-shaped manner around a circumference of separation wall 7. The spaces between ridges 25 are suitable for forming openings 27 (not seen in Fig. 1) of an outer channel 5 to be defined in the assembled sound absorber. Moreover, separation wall 7 and ridges 25 are preferably formed to define a channel 5 extending generally coaxially to the channel 3 in an assembled state of the sound absorber 1. Openings 27 are preferably defined by separation wall 7, ridges 25 and a wall 28. Preferably, separation wall 7 constitutes an inner wall of openings 27 wherein wall 28 constitutes an outer wall. Preferably, wall 28 also supports sound absorbing member 21.

Outer channel member 19 preferably comprises the connector 15, as discussed above, and a sidewall 29 on the side of the connector 15 for forming a sidewall of the sound absorber 1 opposing sidewall 23, as discussed above with regard to inner channel member 17 and the end of connector 15. Sidewall 29 of outer channel means 19 also comprises ridges or separation means 31 extending radially and which are distanced around a radius corresponding to the outer diameter of a corresponding portion of inner channel member 3, preferably corresponding to the outer diameter of separation wall 7 at its end opposite connecting member 13. Between ridges 31, openings 33 (not shown in Fig. 1; see Fig. 3) are provided which form a part of the channel 5. Also channel member 19 further comprises an outer wall portion 35 for defining an outer portion of housing 11 of the sound absorber 1, thereby also forming a sound absorbing volume 9 of the sound absorber 1. Preferably, inner channel member 17 and outer channel member 19 are designed such that the ridges 31 are adapted to fittingly slide over the respective end of the inner channel member 17, here the end of separation wall 7. The ends of outer walls 35 end adjacent sidewalls 23. Preferably, either inner channel member 17 and/or outer channel member 19 comprise means 37 for connecting inner channel member 17 and outer channel member 19. Preferably, means 37 are arranged at the end of either outer wall 35 and/or the ends of sidewalls 23 in order to, preferably, sealingly, close and/or connect inner channel member 17 and outer channel member 19 to form sound absorber 1. Means 37 may preferably comprise snap-fit means and/or thread-means for connecting inner channel member 17 and outer channel member 19.

According to a preferred embodiment, sound absorber 1 further comprises a sound-absorbing member 21 formed to be placed inside the sound absorbing volume 9 established inside the sound absorber 1. Preferably, sound absorbing member 21 is made of, essentially consists of or comprises preferred sound absorbing materials as discussed above. Moreover, sound absorbing member 21 preferably comprises an inner wall 39 defining an outer wall of the outer channel 5. Accordingly, outer channel 5 is preferably defined by an outer wall or separation wall 7, an inner wall or surface 39 of sound absorbing member 21 as well as by openings provided between ridges 25 and 31.

Fig. 3 shows a schematic view of components 17, 19 and 21 which are preferably adapted to be assembled by sliding the components over/into one another. Preferably, components 17, 19 and 21 are fixed to one another to provide a sound absorber 1 by means 37, preferably comprising sealing means as well as, preferably releasable, fastening means. Preferably, the components of the sound absorber 1 can be unfastened or released in order to, e.g., allow a change or disinfection etc. of one or more of the components of the sound absorber 1.

According to a preferred embodiment, wall 23 and ridges 25 etc. are formed as part of inner channel module 17 whereas ridges 31 and wall 29 etc. form part of outer channel module 19.

Sound absorbing member 21 preferably rests on the outer circumference of ridges 25 and 31 provided by the inner channel member 17 and outer channel member 19, respectively.

Preferably, the sound absorber 1 comprises a circular and/or basically rotational symmetrical shape when viewed in a cross-sectional extending basically perpendicular to the axes of channels 3 and/or 5. However, it is understood that also a polygonal or other forms can be suitable for forming the sound absorber and/or inner and outer channel 3, 5.

Figure 4 shows a further schematic sectional view of a sound absorber according to a preferred embodiment of the present invention, preferably according to the embodiment as shown in Figure 1, however, in a cross-sectional view turned around the longitudinal axis of the absorber relative to Figure 1. The same reference numerals refer to corresponding features. In particular, in the cross-sectional view according to Figure 4, the cross-section does not run through ridges 25 but through openings 27 which are formed by separation wall 7 as an inner wall and wall 28 of conduit portion 17 as an outer wall wherein ridges 25 preferably longitudinally extend between separation wall 7 and wall 28 basically in the direction of flow, as indicated by the arrows in Figure 4 or 1. In this cross-sectional view, furthermore openings 33 can be seen which are defined by a separation wall 7, ridges 31 and wall 32. Preferably, the openings 33 are defined by separation wall 7, ridges 31 and wall 32 in the same manner as discussed above with regard to openings 27. Walls 32 preferably also support sound absorbing member 21. Preferably, walls 32 form part of outer channel member 19 whereas walls 28 preferably form part of inner channel module 17.

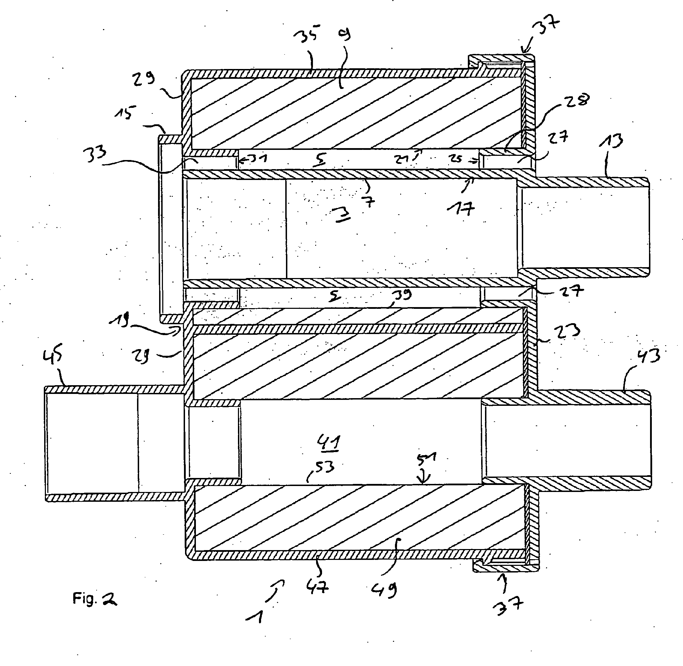

Fig. 2 shows a further preferred embodiment of a sound absorber 1 according to the present invention wherein corresponding reference numerals refer to corresponding features. In particular, sound absorber 1 according to this embodiment additionally comprises a third channel 41 being adapted to for conducting inspiration air from a ventilation device or breathing device (not shown) to a patient. Preferably, channel 41 forms part of an inspiration line or inspiration hose (not shown) of the breathing system or may even be formed by the inspiration hose (not shown). According to the shown preferred embodiment, sound absorber 1 additionally comprises connector means 43 and 45 for connecting to and/or receiving an inspiration hose and/or inspiration connector of a breathing device, respectively, preferably, connector 43 is arranged at the same side of the sound absorber 1 as connector 13 and is defined in or at an extension of sidewall 23 of the inner channel member 17. Connector 45 is preferably arranged at the same side of sound absorber 1 as connector 15 and is preferably formed in or at an extension of the sidewall 29 of outer channel member 19.

Moreover, sound absorber 1 preferably comprises a further outer wall component 47, preferably provided by outer channel means 19, defining a second sound absorbing volume 49 surrounding a third channel 41. Preferably, sound absorbing volume 49 can be filled with a sound absorbing filler medium, as discussed above, preferably constituted by a second sound absorbing member 51. Preferably, an inner wall or surface 53 of the sound absorbing member 51 is adapted to define an outer wall of third channel 41.

Preferably, connecting means 37 are provided between inner channel member 17 and outer channel member 19 at the ends of sidewall 23 and outer walls 35 and 47, respectively.

The provision of third channel 41 additionally reduces and damps the noise conducted from the breathing device through the inspiration hose to the patient.

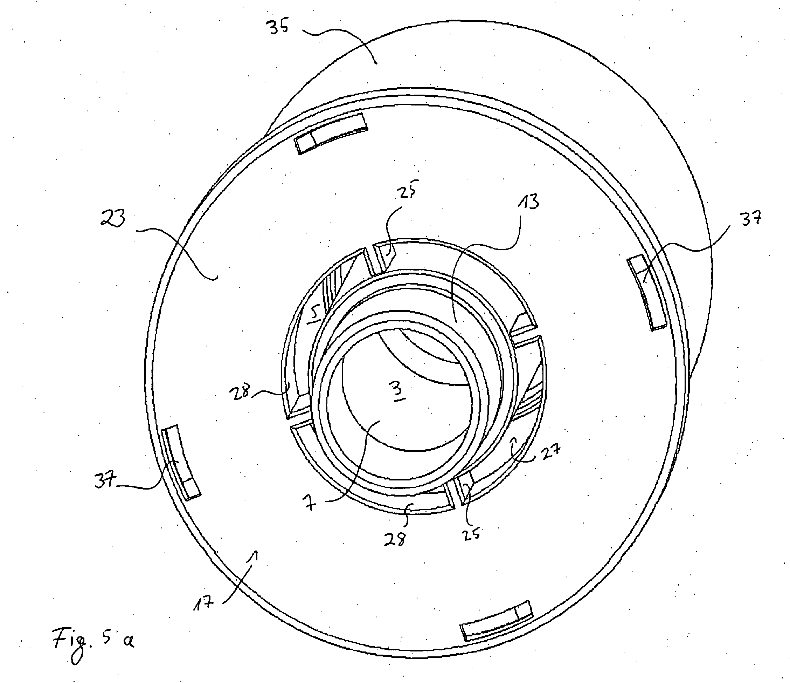

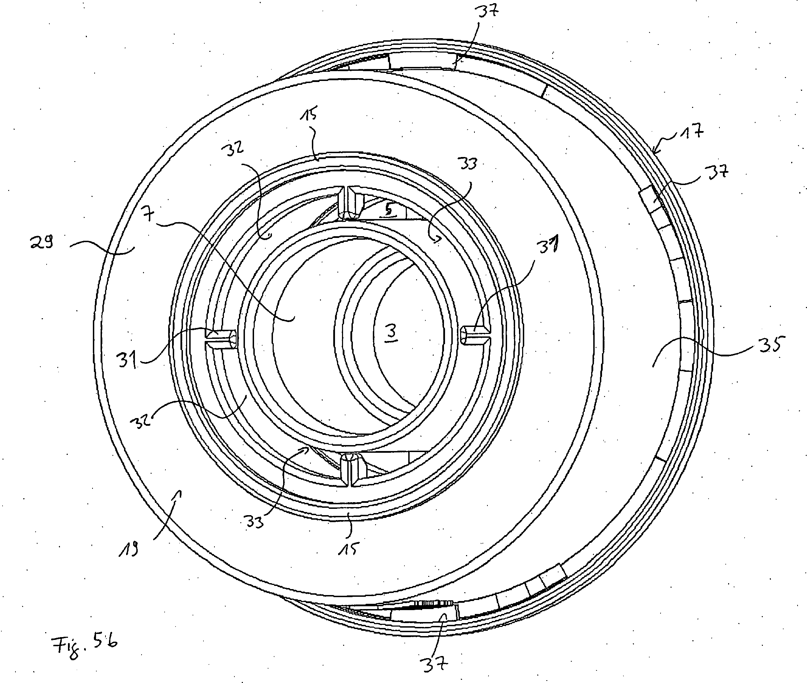

Figure 5a and 5b show three-dimensional views of a sound absorber according to the present invention, preferably according to the sound absorber of the first embodiment, wherein Figure 5a shows a view onto the sound absorber basically from the right side according to the orientation shown in Figure 1 and Figure 5b shows a view from the left side basically according to the orientation of Figure 1.

Since Figures 5a and 5b show corresponding configurations to those shown, e.g., in Figure 1, 3 and 4 and partly also in Figure 2, it is referred to the respective discussion.

In Figure 5a in a conduit portion 17 and a lid or sidewall portion 23 thereof can be seen. Fastening and/or sealing means 37 as correspondingly provided on inner channel module 17 and outer channel module 19 are realized as thread members. In Figure 5a ridges 25 defining, together with separation wall 7 as an inner wall and wall 28 as an outer wall, openings 27. Openings 27 again form part or lead into channel 5 extending generally coaxially to channel 3 as defined inside the preferably circular separation wall 7. In Figure 5b particularly, outer channel member 19 and sidewall 29 can be seen. Thus, Figure 5b shows a view onto the opposite end of sound absorber 1 than Figure 5a. In Figure 5b, clearly ridges 31, defining together with separation wall 7 as an inner wall and wall 32 as an outer wall openings 33 leading into or forming a part of outer channel 5 can be seen. A channel 3 extends preferably coaxial to outer channel 5 inside preferably circular separation wall 7.

The present invention is particularly advantageous in that it provides a simple and effective solution for absorbing sound which is easily adaptable to a number of different designs of breathing systems and ventilation means, has low manufacturing costs and allows an easy cleaning and disinfection or even replacement of its components. Additionally, the device and particularly the channels provided by the sound-absorbing device have a low flow resistance comparable to the flow resistance of the inspiration or expiration hose. Preferably, the sound absorbing members 21 and 51, respectively, can be, preferably separately, exchanged upon contamination. In this context, it is advantageous that the present invention upholds the separation of inspiration hose and expiration hose so that a cross-contamination of the two circuit components can be avoided.

The sound absorbing members can be designed either as being exchangeable or as a one-way product.

The connection 37 between the inner circuit member 17 and the outer circuit member 19 may either be provided by, e.g., a screw or thread connection, a flipping connection and the like and connecting means 37 may be provided with further sealing means.

Optionally, the sound absorber according to the present invention may comprise an integrated humidifier. This can be easily achieved by, e.g., humidifying the sound absorbing means 51. Furthermore, the sound absorbing means 1 may additionally provide a filter medium (not shown) extending through the inspiration and/or expiration channel. Preferably, the respective filter media are separately exchangeable.

When using foam materials as the sound-absorbing member 21 or 51 coming into direct contact with the inspiration air or expiration air, it has to be considered that the addition of O₂ sets the ignition point to a lower temperature.

The connectors 13, 15, 43 and/or 45 can be designed and adapted to provide an easy and reliable connection with the respective hoses, expiration valves and/or ventilation means or breathing devices, depending on the respective requirements.

## Claims

1. Sound absorber (1) for ventilation means having an expiration valve, comprising a first flow channel (3) and a second flow channel (5) for conducting breathable air, wherein the first flow channel (3) and the second flow channel (5) are connectable to the expiration valve so that both air flowing to the expiration valve and air flowing from the expiration valve is directed through one of the first (3) and second (5) flow channels of the sound absorber (1), wherein the first (3) and second (5) flow channels extend substantially co-axial to one another, and wherein the sound absorber comprises a housing (11) providing a sound absorbing volume (9), the housing (11) surrounding the first (3) and second (5) channels.

2. Sound absorber according to claim 1, wherein the first (3) and second (5) flow channels are designed as two co-axially extending tubes.

3. Sound absorber according to any one of the preceding claims, wherein the first channel (3) is inside the second channel (5) or wherein the second channel (5) extends around the first channel (3).

4. Sound absorber according to any one of the preceding claims, wherein the first channel (3) is for conducting exhaled air to the expiration valve and wherein the second channel (5) is for conduction exhaled air from the expiration valve.

5. Sound absorber according to claim 1, wherein the sound absorbing volume (9) is filled with a sound absorbing medium such as air, gas, liquid, fluid, steel, wool, steel wool, sintered materials, porous materials, foam or foamed materials, non-woven materials and woven materials and/or knitted fabrics and the like.

6. Sound absorber according to any one of the preceding claims, wherein the first (3) and second (5) flow channel are distinct from one another.

7. Sound absorber according to any one of the preceding claims, wherein the first flow channel (3) and the second flow channel (5) are separated by a tubular separation wall (7).

8. Sound absorber according to any one of the preceding claims, wherein the sound absorber comprises one opening being directed towards the expiration line of the breathing system as well as two, preferably coaxial, openings for connection to the expiration valve and one opening to the ambience (27).

9. Sound absorber (1) according to any one of the preceding claims, wherein the second flow channel (5) is defined by a separation wall (7) also defining the first flow channel (3) and a sound absorbing volume (9) provided by the sound absorber housing.

10. Sound absorber according to any one of the preceding claims, wherein the sound absorber further comprises a third flow channel (41) for conducting inspiration air from a ventilation means to a patient.

11. Sound absorber according to claim 10, wherein the sound absorber further comprises two additional openings for connection to the respective conduit portions of the inspiration line, i.e., one for connection to the inspiration line and one for connection to the ventilation means.

12. Sound absorber according to any one of the preceding claims, wherein the sound absorber comprises an inner channel module, an outer channel module as well as, preferably, a sound absorbing medium component.

13. Sound absorber according to any one of the preceding claims, the sound absorbing volume being suitable for being filled by a sound absorbing medium component.

14. Expiration valve connected to a sound absorber according to any one of the preceding claims.

15. Method for absorbing sound using a sound absorber according to any of claims 1 to 13 or an expiration valve according to claim 14.

## Patentansprüche

1. Schalldämpfer (1) für eine Ventilierungseinrichtung mit einem Exspirationsventil, der einen ersten Strömungskanal (3) und einen zweiten Strömungskanal (5) zum Durchleiten atembarer Luft aufweist, wobei der erste Strömungskanal (3) und der zweite Strömungskanal (5) mit dem Exspirationsventil verbindbar sind, so dass sowohl zum Exspirationsventil strömende Luft als auch vom Exspirationsventil strömende Luft durch den ersten (3) oder den zweiten (5) Strömungskanal des Schalldämpfers (1) geleitet wird, wobei der erste (3) und der zweite (5) Strömungskanal sich im Wesentlichen koaxial zueinander erstrecken, wobei der Schalldämpfer ein Gehäuse (11) zum Bereitstellen eines schalldämpfenden Volumens (9) aufweist, und wobei das Gehäuse (11) den ersten (3) und den zweiten (5) Strömungskanal umgibt.

2. Schalldämpfer nach Anspruch 1, wobei der erste (3) und der zweite (5) Strömungskanal als zwei sich koaxial erstreckende Rohre konfiguriert sind.

3. Schalldämpfer nach einem der vorangehenden Ansprüche, wobei der erste Kanal (3) im Inneren des zweiten Kanals (5) angeordnet ist oder der zweite Kanal (5) sich um den ersten Kanal (3) herum erstreckt.

4. Schalldämpfer nach einem der vorangehenden Ansprüche, wobei der erste Kanal (3) zum Durchleiten von Ausatmungsluft zum Exspirationsventil dient, und wobei der zweite Kanal (5) zum Durchleiten von Ausatmungsluft vom Exspirationsventil dient.

5. Schalldämpfer nach Anspruch 1, wobei das schalldämpfende Volumen (9) mit einem schalldämpfenden Medium gefüllt ist, wie z.B. mit Luft, einem Gas, einer Flüssigkeit, einem Fluid, Stahl, Wolle, Stahlwolle, Sintermaterialien, porösen Materialien, Schaumstoff oder schaumartige Materialien, Vliesstoffen und Webstoffen und/oder Maschenwaren und Ähnliches.

6. Schalldämpfer nach einem der vorangehenden Ansprüche, wobei der erste (3) und der zweite (5) Strömungskanal einzeln ausgebildet sind.

7. Schalldämpfer nach einem der vorangehenden Ansprüche, wobei der erste (3) und der zweite (5) Strömungskanal durch eine rohrförmige Trennwand (7) getrennt sind.

8. Schalldämpfer nach einem der vorangehenden Ansprüche, wobei der Schalldämpfer eine Öffnung aufweist, die zur Exspirationsleitung des Beatmungssystems hin gerichtet ist, sowie zwei, vorzugsweise koaxiale Öffnungen für eine Verbindung mit dem Exspirationsventil und mit einer Öffnung (27) zur Atmosphäre.

9. Schalldämpfer (1) nach einem der vorangehenden Ansprüche, wobei der zweite Strömungskanal (5) durch eine Trennwand (7), die auch den ersten Strömungskanal (3) definiert, und ein durch das Schalldämpfergehäuse bereitgestelltes schalldämpfendes Volumen (9) definiert ist.

10. Schalldämpfer nach einem der vorangehenden Ansprüche, wobei der Schalldämpfer ferner einen dritten Strömungskanal (41) zum Durchleiten von Inspirationsluft von einer Ventilierungseinrichtung zu einem Patienten aufweist.

11. Schalldämpfer nach Anspruch 10, wobei der Schalldämpfer ferner zwei weitere Öffnungen für eine Verbindung mit jeweiligen Leitungsabschnitten der Inspirationsleitung aufweist, d.h. eine für eine Verbindung mit der Inspirationsleitung und eine für eine Verbindung mit der Ventilierungseinrichtung.

12. Schalldämpfer nach einem der vorangehenden Ansprüche, wobei der Schalldämpfer ein Innenkanalmodul, ein Außenkanalmodul sowie vorzugsweise eine Komponente mit einem schalldämpfenden Medium aufweist.

13. Schalldämpfer nach einem der vorangehenden Ansprüche, wobei das schalldämpfende Volumen dafür geeignet ist, durch eine Komponente mit einem schalldämpfenden Medium gefüllt zu werden.

14. Exspirationsventil, das mit einem Schalldämpfer nach einem der vorangehenden Ansprüche verbunden ist.

15. Verfahren zum Dämpfen von Schall unter Verwendung eines Schalldämpfers nach einem der Ansprüche 1 bis 13 oder eines Exspirationsventils nach Anspruch 14.

## Revendications

1. Amortisseur de bruit (1) pour des moyens de ventilation dotés d'une valve d'expiration, comprenant un premier canal de passage (3) et un second canal de passage (5) destinés à conduire l'air respirable, dans lequel le premier canal de passage (3) et le second canal de passage (5) peuvent être reliés à la valve d'expiration de sorte qu'à la fois l'air passant vers la valve d'expiration et l'air venant de la valve d'expiration soient dirigés vers l'un des premiers (3) et seconds (5) canaux de passage de l'amortisseur de bruit (1), dans lequel les premiers (3) et seconds (5) canaux de passage s'étendent sensiblement coaxialement l'un à l'autre, et dans lequel l'amortisseur de bruit comprend un boîtier (11) fournissant un volume d'absorption de bruit (9), le boîtier (11) entourant les premiers (3) et seconds (5) canaux de passage.

2. Amortisseur de bruit selon la revendication 1, dans lequel les premiers (3) et seconds (5) canaux de passage sont conçus comme deux tubes s'étendant coaxialement.

3. Amortisseur de bruit selon l'une quelconque des revendications précédentes, dans lequel le premier canal (3) est dans le second canal (5) ou dans lequel le second canal (5) s'étend tout autour du premier canal (3).

4. Amortisseur de bruit selon l'une quelconque des revendications précédentes, dans lequel le premier canal (3) est destiné à conduire l'air expiré vers la valve d'expiration et dans lequel le second canal (5) est destiné à conduire l'air expiré venant de la valve d'expiration.

5. Amortisseur de bruit selon la revendication 1, dans lequel le volume d'absorption de bruit (9) est rempli d'un produit d'absorption de bruit tel que de l'air, du gaz, du liquide, du fluide, de l'acier, de la laine, de la laine d'acier, des matériaux frittés, des matériaux poreux, de la mousse ou des matériaux expansés, des matériaux non tissés et des matériaux tissés et/ou des tissus à maille et similaires.

6. Amortisseur de bruit selon l'une quelconque des revendications précédentes, dans lequel les premiers (3) et seconds (5) canaux de passage sont distincts l'un de l'autre.

7. Amortisseur de bruit selon l'une quelconque des revendications précédentes, dans lequel le premier canal de passage (3) et le second canal de passage (5) sont séparés par une paroi de séparation tubulaire (7).

8. Amortisseur de bruit selon l'une quelconque des revendications précédentes, dans lequel l'amortisseur de bruit comprend une ouverture dirigée vers la ligne d'expiration du système respiratoire ainsi que deux ouvertures, de préférence coaxiales, pour la liaison avec la valve d'expiration et une ouverture vers l'environnement (27).

9. Amortisseur de bruit (1) selon l'une quelconque des revendications précédentes, dans lequel le second canal de passage (5) est défini par une paroi de séparation (7) définissant également le premier canal de passage (3) et un volume d'absorption de bruit (9) fourni par le boîtier de l'amortisseur de bruit.

10. Amortisseur de bruit selon l'une quelconque des revendications précédentes, dans lequel l'amortisseur de bruit comprend en outre un troisième canal de passage (41) destiné à conduire l'air d'inspiration des moyens de ventilation vers un patient.

11. Amortisseur de bruit selon la revendication 10, dans lequel l'amortisseur de bruit comprend en outre deux ouvertures supplémentaires pour la liaison avec des parties de conduit respectives de la ligne d'inspiration, c'est-à-dire une ouverture pour la liaison avec la ligne d'inspiration et une ouverture pour la liaison avec les moyens de ventilation.

12. Amortisseur de bruit selon l'une quelconque des revendications précédentes, dans lequel l'amortisseur de bruit comprend un module de canal intérieur, un module de canal extérieur ainsi que de préférence, un composant de produit d'absorption de bruit.

13. Amortisseur de bruit selon l'une quelconque des revendications précédentes, le volume d'amortissement de bruit étant adapté pour être rempli par un composant de produit d'absorption de bruit.

14. Valve d'expiration reliée à un amortisseur de bruit selon l'une quelconque des revendications précédentes.

15. Procédé d'absorption de bruit utilisant un amortisseur de bruit selon l'une quelconque des revendications 1 à 13 ou une valve d'expiration selon la revendication 14.
